Europäisches Patentamt

European Patent Office

Office européen des brevets

(19)

(11) EP 1 410 757 A1

(12) EUROPEAN PATENT APPLICATION

(43) Date of publication:
21.04.2004 Bulletin 2004/17

(51) Int Cl.⁷: $A61B\ 5/022$

(21) Application number: 03016174.9

(22) Date of filing: 16.07.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 16.10.2002 JP 2002301407

(71) Applicant: Colin Corporation
Komaki-shi, Aichi-ken (JP)

(72) Inventor: Narimatsu, Kiyoyuki
Komaki-shi, Aichi-ken (JP)

(74) Representative:
Winter, Brandl, Fürniss, Hübner, Röss, Kaiser,
Polte Partnerschaft
Patent- und Rechtsanwaltskanzlei
Alois-Steinecker-Strasse 22
85354 Freising (DE)

(54) Vital-information obtaining apparatus

(57)     A vital-information obtaining apparatus including a cuff (12) to be worn on a predetermined portion (14) of a living subject, a cuff-pressure control device (50) operable to control an inflation pressure of the cuff, a cuff-pulse-wave detecting device (28) operable to detect a cuff pulse wave which is a pressure pulsation transmitted from the living subject to the cuff; and a vital-information determining device (52, 54) operable while an inflation pressure (PC) of the cuff is held at a value (PCh) higher than a systolic blood pressure of the subject under the control of the cuff-pressure control device, and wherein the vital-information determining means determines vital information of the subject, on the basis of a notch (n) of the cuff pulse wave detected by the cuff-pulse-wave detecting device.

FIG. 2

## Description

This application is based on Japanese Patent Application No. 2002-301407 filed on October 16, 2002, the contents of which are incorporated hereinto by reference.

## BACKGROUND OF THE INVENTION

### Field of the Invention

The present invention relates to a vital-information obtaining apparatus operable to obtain vital or physical information of a living subject or being, on the basis of notches of a pulse wave of a cuff.

### Discussion of Related Art

A blood pressure measuring apparatus using a cuff is widely used, and there have been proposed various types of an apparatus arranged to obtain vital or physical information on a living subject or being, in addition to the blood pressure, by using a cuff pulse wave which is obtained from the cuff. For example, JP-A-2001-346769 describes, on page 10, column 17, lines 3-13, an apparatus arranged to determine a pulse period or a pulse rate from a length of time between predetermined points on the cuff pulse wave, such as a point of a rise and a peak of the wave. JP-B1-3027750 describes, on page 6, column 12, lines 6-28, another type of conventional apparatus, which is arranged to obtain, in addition to a heartbeat-synchronous signal in the form of the cuff pulse wave, another heartbeat-synchronous signal at a portion of the living subject distant from a portion on which the cuff is placed. This type of apparatus obtains pulse-wave-propagating-velocity information relating to a velocity of propagation of the pulse wave through the body of the living subject, such as a pulse-wave propagating velocity and a pulse-wave propagating time.

In the apparatuses disclosed in the above-identified two documents, the cuff pulse wave used to obtain the vital information of the living subject is detected when the inflation pressure of the cuff is lower than the diastolic or minimal blood pressure, in view of a fact that when the cuff inflation pressure is higher than the diastolic blood pressure, the waveform of the cuff pulse wave tends to be distorted and cannot be accurately detected.

However, the cuff pulse wave detected when the cuff inflation pressure is lower than the diastolic blood pressure suffers from indefiniteness in its high-frequency component. The high-frequency component of the pulse wave includes notches, for example. If the notches are used to obtain the vital information such as the pulse-wave propagating velocity, the obtained vital information does not have a sufficiently high degree of reliability.

## SUMMARY OF THE INVENTION

The present invention was made in the light of the background art discussed above. It is therefore an object of the present invention to provide a vital-information obtaining apparatus which is capable of accurately detecting the notches of the pulse wave of the cuff, for obtaining highly reliable vital information of a living subject on the basis of the detected notches.

As a result of extensive studies, the present inventor has found that a cuff pulse wave detected when the cuff inflation pressure is equal to or higher than the systolic blood pressure of the living subject is clearer or more definite in its high-frequency component than a cuff pulse wave detected when the cuff inflation pressure is lower than the diastolic blood pressure, although the former cuff pulse wave is distorted in its low-frequency component due to the relatively high cuff inflation pressure. Namely, the present inventor has found that the notches included in the high-frequency component can be more accurately detected when the cuff pulse wave is detected when the cuff inflation pressure is equal to or higher than the systolic blood pressure, than when the cuff pulse wave is detected when the cuff inflation pressure is lower than the diastolic blood pressure. The present invention was made on the basis of this finding.

The above object may be achieved according to the principle of this invention, which provides a vital-information obtaining apparatus comprising:

a cuff to be worn on a predetermined portion of a living subject;
a cuff-pressure control device operable to control an inflation pressure of the cuff;
a cuff-pulse-wave detecting device operable to detect a cuff pulse wave which is a pressure pulsation transmitted from the living subject to the cuff; and
vital-information determining means operable while an inflation pressure of the cuff is held at a value higher than a systolic blood pressure of the living subject under the control of the cuff-pressure control device, the vital-information determining means determining vital information of the living subject, on the basis of a notch of the cuff pulse wave detected by the cuff-pulse-wave detecting device.

In the vital-information obtaining apparatus of the present invention constructed as described above, the notch of the cuff pulse wave detected by the cuff-pulse-wave detecting device while the inflation pressure of the cuff is held at the value higher than the systolic pressure under the control of the cuff-pressure control means can be clearly detected, and the vital or physical information on the living subject can exactly obtained by the vital-information determining means or device, on the basis of the thus detected notch.

According to a first preferred form of the vital-information obtaining apparatus of the present invention, the vital-information determining means or device determines, as the vital information, an ejection time of a heart of the living subject, on the basis of a time difference between moments at which a rise point and the notch appear in the cuff pulse wave.

According to a second preferred form of the invention, the vital-information obtaining apparatus further comprises a heartbeat-synchronous-signal detecting device to be worn on a portion of the living subject which is not located downstream of the cuff, the heartbeat-synchronous-signal detecting device being operable to detect a heartbeat-synchronous signal which is generated by said living subject and which is synchronized with a heartbeat of the living subject, and wherein the vital-information determining means is operable to determine, as the vital information, pulse-wave propagating-velocity information relating to a velocity of propagation of a pulse wave through the living subject, on the basis of the notch of the cuff pulse wave detected by the cuff-pulse-wave detecting device while the inflation pressure of the cuff is held at the value higher than the systolic blood pressure of the living subject under the control of the cuff-pressure control device, and on the basis of a portion of the heartbeat-synchronous signal detected by the heartbeat-synchronous-signal detecting device when the cuff pulse wave is detected, the above-indicated portion of the heartbeat-synchronous signal corresponding to the notch of the cuff pulse wave.

According to one advantageous arrangement of the second preferred form of the invention, the heartbeat-synchronous-signal detecting device includes a heart-sound microphone operable to detect a moment of initiation of generation of a second heart sound of the living subject, on the basis of a heart-sound wave represented by the heartbeat-synchronous signal, and the vital-information determining means includes pulse-wave-propagating-velocity calculating means for calculating a pulse-wave propagating time between the moment of initiation of generation of the second heart sound detected by the heart-sound microphone, and a moment of generation of the notch of the cuff pulse wave detected by the cuff-pulse-wave detecting device.

In the above-indicated advantageous arrangement, the pulse-wave-propagating-velocity calculating means may be arranged to calculate a propagating distance on the basis of a known height T of the living subject, and the velocity of propagation of the pulse wave through the living subject, on the basis of the propagating distance and the pulse-wave propagating time.

Where the pulse-wave-propagating-velocity calculating means is arranged to calculate the velocity of propagation of the pulse wave, as described above, the vital-information obtaining apparatus may further comprise an input device through which a signal indicative of the known height of the living subject is applied to the pulse-wave-propagating-velocity calculating means.

According to a third preferred form of the vital-information obtaining apparatus, the vital-information determining means determines the vital information on the basis of the notch of the cuff pulse wave while the inflation pressure of the cuff is held at a blood-flow shut-off pressure value at which no blood flow is present through the predetermined portion of the living subject on which said cuff is worn.

BRIEF DESCRIPTION OF THE DRAWINGS

The above and other objects, features, advantages and technical and industrial significance of the present invention will be better understood by reading the following detailed description of a presently preferred embodiment of the invention, when considered in connection with the accompanying drawings, in which:

Fig. 1 is a block diagram showing a circuit arrangement of a vital-information obtaining apparatus constructed according to one embodiment of the present invention;

Fig. 2 is a functional block diagram for explaining major functional portions of an electronic control device of the vital-information obtaining apparatus of Fig. 1;

Fig. 3 is a view indicating a cuff pulse wave detected when a cuff pressure PC is set to be equal to a blood-flow-shut-off pressure value PCh (cuff pulse wave detected at high cuff pressure), together with a cuff pulse wave detected when the cuff pressure value PC is set to be equal to or lower than the diastolic blood pressure (cuff pulse wave detected at low cuff pressure); and

Fig. 4 is a flow chart illustrating a portion of a control operation of a CPU shown in the functional block diagram of Fig. 2.

DETAILED DESCRIPTION OF THE PREFERRED EMBODIMENT

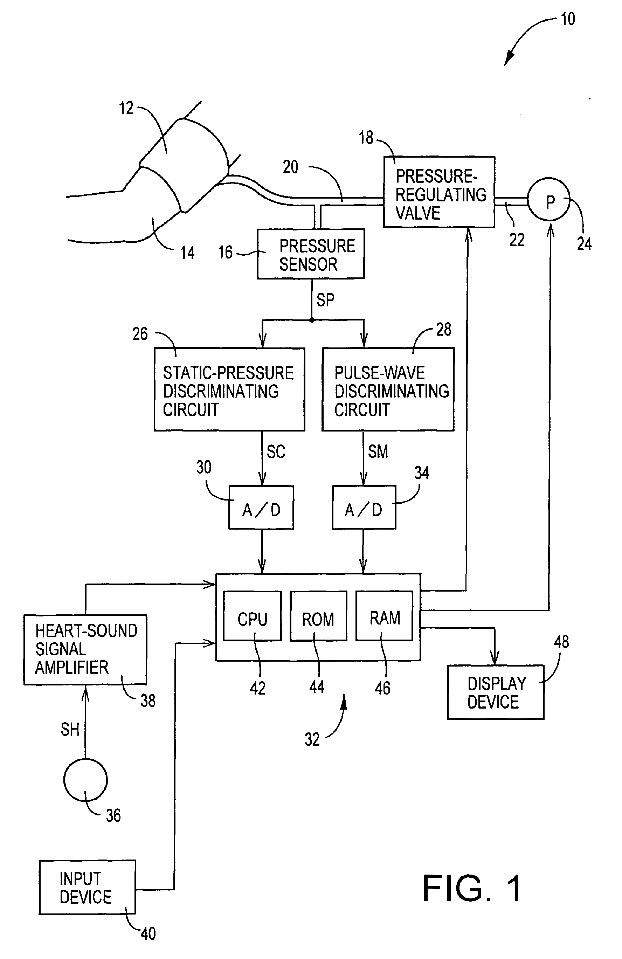

Referring to the drawings, there will be described in detail a preferred embodiment of this invention. The block diagram of Fig. 1 shows a circuit arrangement of a vital-information obtaining apparatus 10 constructed according to one embodiment of the present invention.

The vital-information obtaining apparatus 10 includes a cuff 12 which has a rubber bag accommodated in an elongate fabric bag. The cuff 12 is arranged to be wound or worn on an upper arm (brachium) 14 of a living subject. To this cuff 12, there are connected a pressure sensor 16 and a pressure regulating valve 18 through a conduit 20. The pressure regulating valve 18 is connected to an air pump 24 through a conduit 22. The pressure regulating valve 18 is provided to regulate the pressure of an air pressurized by the air pump 24 and apply the thus regulated air pressure to the cut 12, and to permit the pressurized air to be discharged from the cuff 12, for thereby regulating the air pressure in the cuff 12, that is, an inflation pressure of the cuff 12.

The pressure sensor 16 is arranged to detect the air pressure in the cuff 12, and apply a pressure signal SP indicative of the detected air pressure to a static-pressure discriminating circuit 26 and a pulse-wave discriminating circuit 28. The static-pressure discriminating circuit 26 includes a low-pass filter and obtains a cuff-pressure signal SC indicative of a steady pressure component included in the pressure signal SP, that is, an inflation pressure of the cuff 12 (hereinafter referred to as "cuff pressure PC"). The cuff-pressure signal SC is applied to an electronic control device 32 through an A/D converter 30. The pulse-wave discriminating circuit 28 includes a band-pass filter operable to transmit a signal within a predetermined frequency band, for instance, within a frequency band between about 1Hz and about 30Hz, for obtaining a cuff-pulse-wave signal SM indicative of an oscillating component of the pressure signal SP. The cuff-pulse-wave signal SM is applied to the electronic control device 32 through an A/D converter 34. This cuff-pulse-wave signal SM represents a pressure pulsation transmitted from an arterial vessel of the subject to the cuff 12, that is, a pulse wave of the cuff 12 (hereinafter referred to as "cuff pulse wave"). Thus, the pulse-wave discriminating circuit 28 functions as a cuff-pulse-wave detecting device operable to detect the cuff pulse wave. In the present embodiment, the arterial vessel indicated above is the arterial vessel of one of the upper arms (brachia) of the subject, and the cuff pulse wave represents a pulse wave of the arterial vessel of the upper arm.

The present vital-information obtaining device 10 further includes a heart-sound microphone 36, which functions as a heartbeat-synchronous-signal detecting device. The heart-sound microphone 36 is fixed on the chest (thoracic region) of the subject (not shown), with an adhesive tape, for instance. The heart-sound microphone 36 incorporates a piezoelectric element operable to convert a heart sound (cardinal sound) generated by the heart of the subject, into an electric signal, that is, a heartbeat-synchronous signal in the form of a heart-sound signal SH synchronized with the heartbeat of the subject. The heart-sound microphone 36 is connected to a heart-sound signal amplifier 38, which is provided with four kinds of filters (not shown) operable to amplify a high-pitch component of the heart sound while attenuating a low-pitch component of the heart sound which has a relatively large energy. The heart-sound signal amplifier 38 receives the heart-sound signal SH, and performs amplifying and filtering operations to process the received heart-sound signal SH. An output of the heart-sound signal amplifier 38 is applied to the electronic control device 32 through an A/D converter (not shown).

The vital-information obtaining apparatus 10 further includes an input device 40, which is provided with a keyboard (not shown) through which a height T of the subject is entered. A height signal ST indicative of the entered height T of the subject is applied to the electronic control device 32.

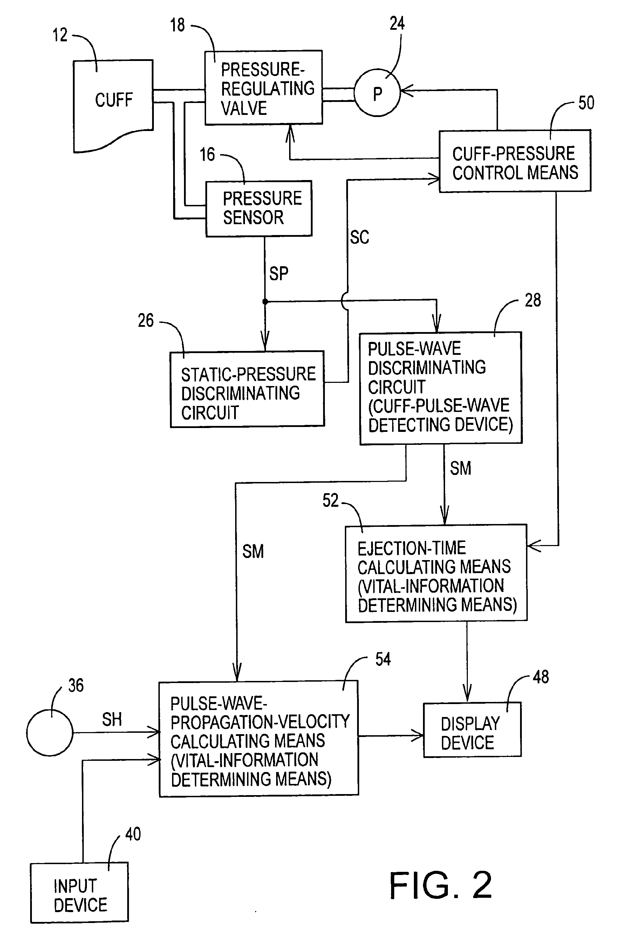

The electronic control device 32 is constituted by a so-called microcomputer incorporating a CPU (central processing unit) 42, a ROM (read-only memory) 44, a RAM (random-access memory) 46, and an I/O (input/output) port (not shown). The CPU 42 operates according to control programs stored in the ROM 44 while utilizing a data storage function of the RAM 46, to perform signal processing operations and apply drive signals to the air pump 24 and the pressure regulating valve 18 through the I/O port, for controlling the air pump 24 and the pressure regulating valve 18, so as to control the cuff pressure PC. The CPU 42 performs other functions illustrated in detail in the functional block diagram of Fig. 2, to calculate an ejection time ET and a pulse-wave propagating velocity PWV, and to control a display 48.

Referring to the functional block diagram of Fig. 2, there are illustrated major functional portions of the electronic control device 32 in the vital-information obtaining apparatus 10.

The electronic control device 50 incorporates a cuff-pressure control means or portion 50, an ejection-time calculating means or device 52, and a pulse-wave-propagating-velocity calculating means or device 54. The cuff-pressure control means 50 is arranged to determine the cuff pressure PC on the basis of the cuff-pressure signal SC received from the static-pressure discriminating circuit 26, and controls the pressure regulating valve 18 and the air pump 24, to control the cuff pressure PC to a predetermined blood-flow shut-off pressure value PCh (e.g., 180mmHg) which is higher than the systolic pressure of the subject. In the present vital-information obtaining apparatus 10, the static-pressure discriminating circuit 26, pressure regulating valve 18, air pump 24 and cuff-pressure control means or portion 50 cooperate to constitute a cuff-pressure control device.

The ejection-time calculating means or device 52, which functions as a part of vital-information determining means or device, is operable while the cuff pressure PC is held at the predetermined blood-flow shut-off pressure value PCh under the control of the cuff-pressure control device 50. In this condition, the ejection-time calculating means 52 calculates a time difference or length between a moment of detection of a rise point "s" of the cuff pulse wave represented by the cuff-pulse-wave signal SM generated from the pulse-wave discriminating circuit 28, and a moment of detection of a notch "n" of the cuff pulse wave. The ejection-time calculating means 52 calculates the above-indicated time difference or length, as an ejection time ET of the heart of the subject, and controls the display device 48 so as to indicate the calculated ejection time ET. The ejection time ET is a length of time from a moment at which ejection of the blood from the left ventricle of the heart is initiated with the aortic valve in its open state, to a moment at which the aortic valve is closed. The rise point "s" appears at the moment of initiation of the blood ejection upon opening of the aortic valve, while the notch "n" appears at the moment of closure of the aortic valve. Therefore, the length of time between the moments of detection of the rise point "s" and the notch "n" represents the ejection time (left ventricular ejection time) ET.

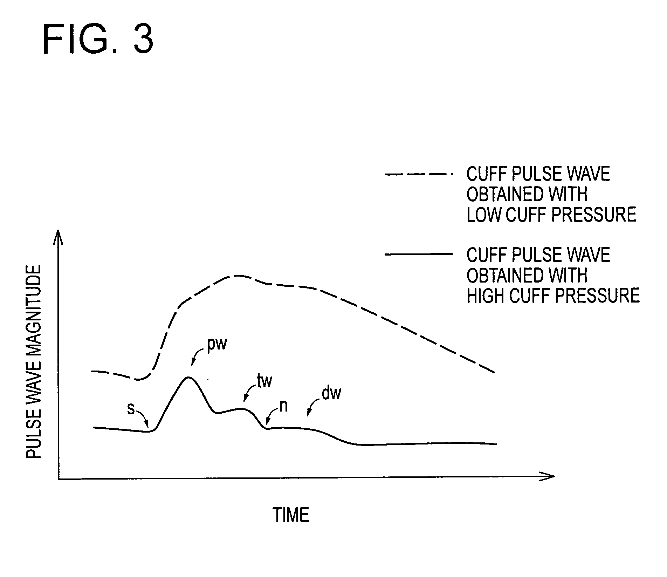

There will next be described the cuff pulse wave as detected while the cuff pressure PC is held at the blood-flow shut-off pressure value PCh. Fig. 3 is a view indicating a cuff pulse wave detected when the cuff pressure PC is set to be equal to the blood-flow-shut-off pressure value PCh (cuff pulse wave detected at high cuff pressure), together with a comparative cuff pulse wave detected when the cuff pressure PC is set to be equal to or lower than the diastolic blood pressure (cuff pulse wave detected at low cuff pressure) as in the prior art. When the cuff pressure PC is held at the blood-flow shut-off pressure value PCh, no blood flow is present under the cuff 12. The cuff pulse wave at the high cuff pressure equal to the blood-blow shut-off pressure value PCh represents a pulsation which is generated at a portion of the upper arm 14 upstream of the cuff 12 and which is transmitted to the upstream end of the cuff 12. Accordingly, the cuff pulse wave at the high cuff pressure has a smaller magnitude as a whole, than the cuff pulse wave at the low cuff pressure not higher than the diastolic blood pressure, but has two peaks that can be clearly detected. These two peaks correspond to a percussion wave "pw" and a tidal wave "tw". While the percussion wave "pw" and the tidal wave "tw" cannot be clearly discriminated in the cuff pulse wave at the low cuff pressure, the high-frequency component including those percussion wave "pw" and tidal wave "tw" can be detected more clearly and prominently in the cuff pulse wave at the high cuff pressure, than the low-frequency component. On the other hand, the notch "n" appears between the tidal wave "tw" and the following dicrotic wave "dw", and can be detected as a first minimal point after the peak in the form of the tidal wave "tw".

The pulse-wave-propagating-velocity calculating means or device 54, which functions as a part of the vital-information determining means or device, is also operable while the cuff pressure PC is held at the blood-flow shut-off pressure value PCh under the control of the cuff-pressure control means 50. In this condition, the pulse-wave-propagating-velocity calculating means 54 calculates a time difference or length (sec) between a moment at which a second heart sound is initially detected based on a heart-sound wave by the hear-sound microphone 36, and a moment at which the notch "n" of the cuff pulse wave is detected by the pulse-wave discriminating circuit 28. Like the notch "n", the second heart sound is generated due to closure of the aortic valve, so that the detected time difference indicated above represents a pulse-wave propagating time DT, which is a time duration of propagation of the pulse wave from the heart to a cuff-worn portion of the upper arm 14 on which the cuff 12 is worn.

The pulse-wave-propagating-velocity calculating means 54 is further arranged to obtain a propagating distance L between the heart and the cuff-worn portion, on the basis of the height T of the subject received from the input device 40, and according to a predetermined equation (1) representative of a predetermined relationship between the height T and the propagating distance L. The pulse-wave-propagating-velocity calculating means 54 then calculates a pulse-wave-propagating velocity PWV (cm/sec) on the basis of the obtained propagating distance L and the pulse-wave-propagating time DT indicated above, and according to a predetermined equation (2). The pulse-wave-propagating-velocity calculating means 54 commands the display device 48 to indicate the calculated pulse-wave-propagating velocity PWV.

$$(\text{Equation 1}) \qquad L = aT + b$$

wherein "a" and "b" are experimentally determined constants.

$$(\text{Equation 2}) \qquad PWV = L/DT$$

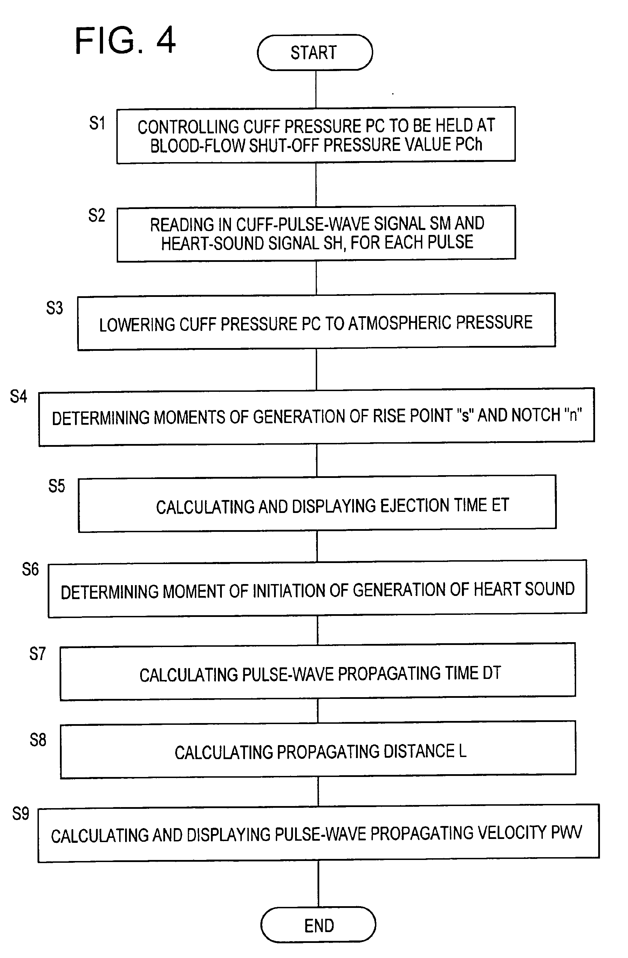

Referring to the flow chart of Fig. 4, there is illustrated a portion of a control operation of the CPU 42 shown in the functional block diagram of Fig. 2. This portion of the control operation is initiated when a start pushbutton (not shown) is depressed after the electronic control device 32 has received the height signal ST from the input device 40.

The control operation of Fig.4 is initiated with step S1 in which the air pump 24 is started, and the pressure regulating valve 18 is controlled to control the cuff pressure PC to the predetermined blood-blow shut-off pressure value PCh, for example 180mmHg.

The control flow then goes to step S2 to read in the cuff-pulse-wave signal SM from the pulse-wave discriminating circuit 28 and the heart-sound signal SH from the heart-sound microphone 36, for one pulse, while the cuff pressure PC is held at the blood-flow shut-off pressure value PCh. After the cuff-pulse-wave signal SM and the heart-sound signal SH have been read in, step S3 is implemented to lower the cuff pressure PC to the atmospheric pressure. It will be understood that a portion of the CPU 42 assigned to implement steps S1 and S3 in Fig. 4 corresponds to the cuff-pressure control means or portion 50.

Step S4 is then implemented to determine the moments at which the rise point "s" and the notch "n" are generated in the cuff pulse wave represented by the cuff-pulse-wave signal SM read in step S2. Step S4 is followed by step S5 to calculate, as the ejection time ET, the time difference between the determined moments of generation of the rise point "s" and the notch "n". The calculated ejection time ET is displayed on the display device 48. It will be understood that a portion of the CPU 42 assigned to implement steps S4 and S5 in Fig. 4 corresponds to the ejection-time calculating means or device 54.

Step S6 is then implemented to determine a moment of initiation of generation of the second heart sound of the living subject, on the basis of the heart-sound wave represented by the heart-sound signal SH read in step S2. Then, the control flow goes to step S7 to calculate the pulse-wave propagating time DT, which is the time difference between the moment of initiation of generation of the second heart sound determined in step S6, and the moment of generation of the notch "n" determined in step S4.

The control flow then goes to step S8 to calculate the propagating distance L on the basis of the height T of the subject represented by the already received height signal ST, and according to the above-indicated equation (1), and then goes to step S9 to calculate the pulse-wave propagating velocity PWV on the basis of the propagating distance L calculated in step S8 and the pulse-wave propagating time DT calculated in step S7, and according to the above-indicated equation (2), and to display the calculated pulse-wave propagating velocity PWV on the display device 48. It will be understood that a portion of the CPU 42 assigned to implement steps S4 and S6-S9 corresponds to the pulse-wave-propagating-velocity calculating means or device 54.

In the embodiment which has been described, the notch "n" of the cuff pulse wave obtained by the pulse-wave discriminating circuit 28 while the inflation pressure of the cuff 12 is held at the blood-flow shut-off pressure value PCh higher than the systolic pressure under the control of the cuff-pressure control means 50 (steps S1 and S3) can be clearly detected, and the ejection-time calculating means 52 (steps S4 and S5) and the pulse-wave-propagating-velocity calculating means 54 (steps S4 and S6-S9) can exactly obtain the ejection time ET and the pulse-wave propagating velocity PWV, on the basis of the thus detected notch "n".

While the preferred embodiment of this invention has been described in detail by reference to the drawings, it is to be understood that the present invention may be otherwise embodied.

For instance, the blood pressure of the subject can be measured by using the cuff 12. In this instance, the blood-flow shut-off pressure value PCh may be determined on the basis of the systolic pressure of the subject measured with the cuff 12.

While the illustrated embodiment uses the heart-sound microphone 36 placed on the thoracic region of the subject, as a heartbeat-synchronous-signal detecting device, the type of the heartbeat-synchronous-signal detecting device and the portion of the subject on which this detecting device is placed are not limited to those of the illustrated embodiment. For example, the heartbeat-synchronous-signal detecting device may be placed on a suitable portion (e.g., wrist) of one of the two arms on which the cuff 12 is not worn, and may be a pressure wave sensor (namely, a so-called "tonometry sensor") which is held in contact with the subject under a suitable pressure, or a device arranged to detect an electrocardiogram (ECG).

It is further to be understood that various other changes may be made in the invention, without departing from the spirit of the present invention.

**Claims**

1. A vital-information obtaining apparatus **characterized by** comprising:

   a cuff (12) to be worn on a predetermined portion (14) of a living subject;
   a cuff-pressure control device (50) operable to control an inflation pressure of said cuff;
   a cuff-pulse-wave detecting device (28) operable to detect a cuff pulse wave which is a pressure pulsation transmitted from the living subject to said cuff; and
   vital-information determining means (52, 54) operable while an inflation pressure (PC) of said cuff is held at a value (PCh) higher than a systolic blood pressure of said living subject under the control of said cuff-pressure control device, said vital-information determining means determining vital information of said living subject, on the basis of a notch (n) of said cuff pulse wave detected by said cuff-pulse-wave detecting device.

2. A vital-information obtaining apparatus according to claim 1, **characterized in that** said vital-information determining means (52, 54) determines, as said vital information, an ejection time (ET) of a heart of the living subject, on the basis of a time difference between moments at which a rise point (s) and said notch (n) appear in said cuff pulse wave.

3. A vital-information obtaining apparatus according to claim 1, **characterized by** further comprising a heartbeat-synchronous-signal detecting device (36) to be worn on a portion of said living subject which is not located downstream of said cuff, said heartbeat-synchronous-signal detecting device being operable to detect a heartbeat-synchronous signal (SH) which is generated by said living subject and which is synchronized with a heartbeat of the living subject,

   and wherein said vital-information determining means (52, 54) is operable to determine, as said vital information, pulse-wave propagating-velocity information relating to a velocity of propagation of a pulse wave through said living subject, on the basis of said notch (n) of said cuff pulse wave detected by said cuff-pulse-wave detecting device (28) while said inflation pressure of said cuff is held at said value (PCh) higher than the systolic blood pressure of said living subject under the control of said cuff-pressure control device, and on the basis of a portion of said heartbeat-synchronous signal detected by said heartbeat-synchronous-signal detecting device when said cuff pulse wave is detected, said portion of said heartbeat-synchronous signal corresponding to said notch of said cuff pulse wave.

4. A vital-information obtaining apparatus according to claim 3, **characterized in that** said heartbeat-synchronous-signal detecting device includes a heart-sound microphone (36) operable to detect a moment of initiation of generation of a second heart sound of the living subject, on the basis of a heart-sound wave represented by said heartbeat-synchronous signal (SH), and said vital-information determining means (52, 54) includes pulse-wave-propagating-velocity calculating means (54) for calculating a pulse-wave propagating time (DT) between said moment of initiation of generation of said second heart sound detected by said heart-sound microphone, and a moment of generation of said notch (n) of said cuff pulse wave detected by said cuff-pulse-wave detecting device (28).

5. A vital-information obtaining apparatus according to claim 4, **characterized in that** said pulse-wave-propagating-velocity calculating means is operable to calculate a propagating distance (L) on the basis of a known height T of the living subject, and said velocity of propagation of said pulse wave through said living subject, on the basis of said propagating distance and said pulse-wave propagating time (DT).

6. A vital-information obtaining apparatus according to claim 5, **characterized by** further comprising an input device (40) through which a signal indicative of said known height (T) of the living subject is applied to said pulse-wave-propagating-velocity calculating means.

7. A vital-information obtaining apparatus according to any one of claims 1-6, **characterized in that** said vital-information determining means determines said vital information on the basis of said notch (n) of said cuff pulse wave while said inflation pressure (PC) of said cuff (12) is held at a blood-flow shut-off pressure value (PCh) at which no blood flow is present through said predetermined portion of said living subject on which said cuff is worn.

EP 1 410 757 A1

FIG. 1

FIG. 2

# FIG. 3

# FIG. 4

START

S1 | CONTROLLING CUFF PRESSURE PC TO BE HELD AT BLOOD-FLOW SHUT-OFF PRESSURE VALUE PCh

S2 | READING IN CUFF-PULSE-WAVE SIGNAL SM AND HEART-SOUND SIGNAL SH, FOR EACH PULSE

S3 | LOWERING CUFF PRESSURE PC TO ATMOSPHERIC PRESSURE

S4 | DETERMINING MOMENTS OF GENERATION OF RISE POINT "s" AND NOTCH "n"

S5 | CALCULATING AND DISPLAYING EJECTION TIME ET

S6 | DETERMINING MOMENT OF INITIATION OF GENERATION OF HEART SOUND

S7 | CALCULATING PULSE-WAVE PROPAGATING TIME DT

S8 | CALCULATING PROPAGATING DISTANCE L

S9 | CALCULATING AND DISPLAYING PULSE-WAVE PROPAGATING VELOCITY PWV

END

**European Patent Office**

**EUROPEAN SEARCH REPORT**

Application Number

EP 03 01 6174

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| P,X | EP 1 264 573 A (COLIN CORP) 11 December 2002 (2002-12-11) | 1,2,7 | A61B5/022 |
| A | * paragraphs [0036],[0051] * | 3 | |
| X | NISHIYASU T ET AL: "Enhancement of parasympathetic cardiac activity during activation of muscle metaboreflex in humans." JOURNAL OF APPLIED PHYSIOLOGY (BETHESDA, MD.: 1985) UNITED STATES DEC 1994, vol. 77, no. 6, December 1994 (1994-12), pages 2778-2783, XP008024740 ISSN: 8750-7587 | 1,2 | |
| Y | page 2779, left-hand column, line 17 - right-hand column, line 18 | 3-6 | |
| Y | NARIMATSU K ET AL: "A MULTI-ELEMENT CAROTID TONOMETRY SENSOR FOR NON-INVASIVE MEASUREMENT OF PULSE WAVE VELOCITY" FRONTIERS OF MEDICAL AND BIOLOGICAL ENGINEERING, VSP. ZEIST, NL, vol. 11, no. 1, 2001, pages 45-58, XP001095206 ISSN: 0921-3775 * abstract * | 3-6 | TECHNICAL FIELDS SEARCHED (Int.Cl.7) A61B |
| A | US 5 316 004 A (CHESNEY CHARLES F ET AL) 31 May 1994 (1994-05-31) * column 4, line 44 - line 65 * | 1 | |

The present search report has been drawn up for all claims

| Place of search | Date of completion of the search | Examiner |
|---|---|---|
| THE HAGUE | 18 November 2003 | Knüpling, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 03 01 6174

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2003

| Patent document cited in search report | | Publication date | Patent family member(s) | | Publication date |
|---|---|---|---|---|---|
| EP 1264573 | A | 11-12-2002 | JP | 2002360526 A | 17-12-2002 |
| | | | EP | 1264573 A2 | 11-12-2002 |
| | | | US | 2002188209 A1 | 12-12-2002 |
| US 5316004 | A | 31-05-1994 | US | 5211177 A | 18-05-1993 |
| | | | US | 6048318 A | 11-04-2000 |
| | | | US | 6290651 B1 | 18-09-2001 |
| | | | US | 5876347 A | 02-03-1999 |
| | | | US | 2002028998 A1 | 07-03-2002 |
| | | | AU | 9092291 A | 17-08-1992 |
| | | | DE | 69131717 D1 | 18-11-1999 |
| | | | DE | 69131717 T2 | 08-06-2000 |
| | | | EP | 0564492 A1 | 13-10-1993 |
| | | | HK | 1014351 A1 | 16-06-2000 |
| | | | WO | 9211805 A1 | 23-07-1992 |

EPO FORM P0459

For more details about this annex : see Official Journal of the European Patent Office, No. 12/82